# EUROPEAN PATENT SPECIFICATION

(11) **EP 4 473 896 B1**
(45) Date of publication and mention of the grant of the patent: **18.11.2026**
(21) Application number: 24208615.5
(22) Date of filing: 12.03.2019
(51) Int. Cl.: A61B 8/12, A61B 8/00, A61B 1/00, A61B 10/02, A61B 1/31, A61B 8/08

(54) **ULTRASOUND-GUIDED TRANSPERINEAL AND/OR TRANSRECTAL BIOPSY**
ULTRASCHALLGEFÜHRTE TRANSPERINALE UND/ODER TRANSREKTALE BIOPSIE
BIOPSIE TRANSPÉRINÉALE ET/OU TRANSRECTALE GUIDÉE PAR ULTRASONS

(43) Date of publication of application: 11.12.2024
(62) Document number(s) of the earlier application(s) in accordance with Art. 76 EPC: 19724227.4 / 3 937 792
(73) Proprietor: B-K Medical ApS, 2730 Herlev (DK)
(72) Inventor: ROSSEN, Rasmus Holdensgaard, Vanlose (DK); NIELSEN, Torben Svanberg, Kobenhavn S. (DK); HESTBEK, Mai, Kobenhavn (DK); GRAN, Fredrik, Limhamn (SE); JENSEN, Henrik, Nordhavn (DK)
(74) Representative: Gevers Patents

(56) References cited:
- WO-A1-2017/007875
- US-A- 4 742 829
- US-A1- 2015 230 690
- US-A1- 2015 282 880
- US-A1- 2018 325 602

## Description

### TECHNICAL FIELD

The following generally relates to ultrasound imaging and more particularly to ultrasound-guided transperineal and/or transrectal biopsy.

### BACKGROUND

Ultrasound imaging has provided useful information about the interior characteristics of an object or subject under examination. One application includes ultrasound-guided (transrectal or transperineal) prostate biopsy. Generally, a prostate biopsy is a procedure to remove a tissue sample(s) from a suspect area of the prostate, e.g., to rule out / diagnose cancer.

With a transrectal prostrate biopsy procedure, the ultrasound imaging probe and the biopsy instrument are inserted into the rectum via the anus and the biopsy instrument is used to acquire a sample(s) from the prostrate through the rectal wall. With a transperineal prostrate biopsy procedure, the ultrasound imaging probe is inserted into the rectum via the anus and the biopsy instrument is used to acquire a sample(s) from the prostrate through the perineum, which is the region behind the scrotum and in front of the anus. In both instance, ultrasound imaging is used to guide the biopsy instrument to the target area of the prostrate. A trend has been to perform transperineal biopsies, e.g., because of the risk of infection due to puncturing the rectal wall along with antibiotic resistant bacteria with transrectal biopsies.

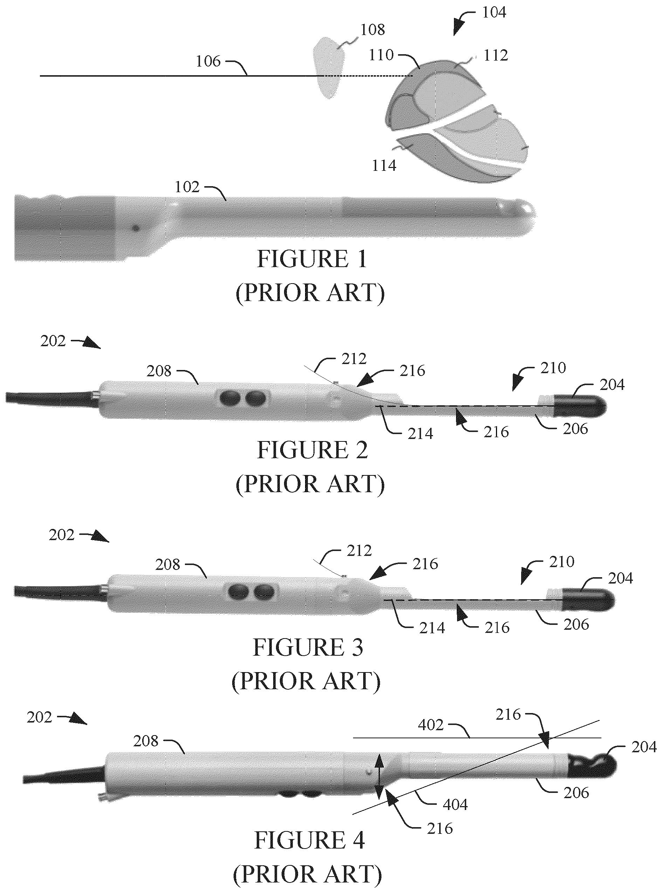

Transperineal biopsies have been performed with either a parallel trajectory or a fan / angled trajectory, using either a physical guide or free-hand. Figure 1 shows an example diagram of a side view of a portion of an ultrasound probe 102 in connection with a sagittal plane of a prostate 104 along with a parallel biopsy needle trajectory 106. As shown, unfortunately, the parallel biopsy needle trajectory 106 can experience physical interference from a pubic archbone 108, limiting access to an apex 110 of an anterior region 112 of the prostate 104. An angled biopsy needle trajectory may be able to reach the apex 110, however, the biopsy instrument may puncture the rectal wall when sampling a peripheral region 114, e.g., because of the combination of the extent of the biopsy instrument and poor visualization of the needle in the ultrasound image.

Figures 2 and 3 show examples of free-hand transrectal biopsies. Both figures show a top-down view of a probe 202, which includes a head 204, a shaft 206, a handle 208, and a cut-out 210 in the shaft 206 to provide space for a needle 212 to be advanced along a center line 214 of the probe 202. In Figure 2, the needle 212 is advanced from above the probe 202 and along a top side 216 of the shaft 206. In Figure 3, the needle 212 is advanced from below the probe 202 up through the cut-out 210 and along the top side 216 of the shaft 206. Unfortunately, these approaches are inflexible in that the range of possible trajectories is limited at least because a portion 216 of the handle 208 is physically in the way and prevents certain trajectories. Figure 4 shows a side view of the probe 202 of Figures 2 and 3, where the portion 216 prevents trajectories between a trajectory 402 (for Figure 2) and a trajectory 404 (for Figure 3).

Transperineal biopsies performed with a guide have used a stationary guide that is in firm contact with the probe so that a needle path can be determined *a priori* and superimposed on an ultrasound image and displayed for tracking and guidance. US 2015/0282880 A1 and US 2016/0022309 A1 discuss setups where the needle is in firm contact with the probe, and US 2011/0009748 A1 discusses a transperineal biopsy that utilizes a guide with predetermined holes for only parallel trajectories. Unfortunately, these approaches constrain how the biopsy is acquired. That is, the biopsy trajectories are constrained to the parallel paths determined by the predetermined guide holes and shown on the displayed image of the predetermined needle paths.

Related technologies are disclosed in US 2015/282880 A1, US 2018/325602 A1, and WO 2017/007875 A1.

US 2015/282880 A1 relates to a system for planning and performing a guided and free-handed transperineal prostate biopsy includes a transrectal ultrasound probe, an access needle configured to perforate a perineal access site of a patient, a biopsy gun, and a guide. The guide includes a sliding platform, stabilization bars, upper and lower mounts, and fasteners. The system and guide apparatus is used for locating a target area using the ultrasound probe, positioning the ultrasound and the access needle at respective designated points, precisely measuring the distance to a designated point, and obtaining specimens from a precise point in the prostate, wherein the method is performed free-handed, and multiple tissue or cell specimens may be obtained from the prostate through an initial access needle.

US 2018/325602 A1 relates to a transperinealprostate intervention device comprises a prostate intervention instrument, a transrectal ultrasound (TRUS) probe, and a mechanical or optical coordinate measurement machine (CMM) attached to the TRUS probe and configured to track the prostate intervention instrument. The CMM may include an articulated arm with a plurality of encoding joints, an anchor end attached to the TRUS probe, and a movable end attached to the prostate intervention instrument. The prostate intervention instrument may, for example, be a biopsy needle, a brachytherapy seed delivery instrument, a tissue ablation instrument, or a hollow cannula. An electronic processor computes a predicted trajectory of the prostate intervention instrument in a frame of reference of the TRUS probe using the CMM attached to the TRUS probe. A representation of the predicted trajectory is superimposed on a prostate ultrasound image generated from ultrasound data collected by the TRUS probe.

WO 2017/007875 A1 relates to a combined imaging probe holder and interventional needle guide assembly is provided. The imaging probe holder supports an imaging device (e.g., ultrasound probe) in a known relationship with a biopsy/treatment device holder such that a delivery element or interventional needle is constrained within an imaging field of the imaging device. Constraining the biopsy treatment device within the imaging field allows for real-time monitoring of the biopsy/treatment device during insertion of the interventional needle into patient tissue. In addition, the biopsy/treatment device holder is angularly positionable relative to the image field to allow advancement of the delivery element to any desired location within the imaging field.

### SUMMARY

The present invention is set out in the appended claims.

### BRIEF DESCRIPTION OF THE DRAWINGS

The application is illustrated by way of example and not limited by the figures of the accompanying drawings, in which like references indicate similar elements and in which:
Figure 1 illustrates a prior art example of a parallel biopsy needle trajectory for a prostrate biopsy procedure;
Figure 2 illustrates a top down view of prior art parallel biopsy needle trajectory advanced from a top of a probe;
Figure 3 illustrates a top down view of prior art angled biopsy needle trajectory advanced from a bottom of a probe up to and along the top of the probe;
Figure 4 illustrates a side view of the probe of Figures 2 and 3, including a region that limits a range of possible biopsy needle trajectories;
Figure 5 diagrammatically illustrates an example ultrasound system in connection with a biopsy instrument in accordance with an embodiment(s) herein;
Figure 6 diagrammatically illustrates a side view of an example biopsy needle guide configured with both parallel and angled trajectory paths coupled to a probe in accordance with an embodiment(s) herein;
Figure 7 diagrammatically illustrates a side view of another example biopsy needle guide configured with both parallel and angled trajectory paths coupled to a probe in accordance with an embodiment(s) herein;
Figure 8 diagrammatically illustrates a top-down view of the example biopsy needle guides of Figure 6 and/or 7 in accordance with an embodiment(s) herein;
Figure 9 diagrammatically illustrates a side view of yet another example biopsy needle guide at a first position which provides a parallel trajectory in accordance with an embodiment(s) herein;
Figure 10 diagrammatically illustrates the side view with the biopsy needle guide at another position which provides a parallel trajectory in accordance with an embodiment(s) herein;
Figure 11 diagrammatically illustrates the side view with the biopsy needle guide at a position which provides an angled trajectory in accordance with an embodiment(s) herein;
Figure 12 diagrammatically illustrates a side view of another example biopsy needle guide that includes a mechanical arm in accordance with an embodiment(s) herein;
Figure 13 diagrammatically illustrates a side view of another example biopsy needle guide that includes a block with predetermined parallel and angled biopsy trajectory channels in accordance with an embodiment(s) herein;
Figure 14 diagrammatically illustrates a perspective view of yet another example biopsy needle guide that includes a column of parallel biopsy trajectory paths and a column of angled biopsy trajectory paths in accordance with an embodiment(s) herein;
Figure 15 diagrammatically illustrates a perspective view of still another example biopsy needle guide that includes a single column of biopsy trajectory paths wherein each path includes a parallel branch and an angled branch in accordance with an embodiment(s) herein;
Figure 16 illustrates a top down view of a probe configured such that its handle does not interfere with biopsy needle trajectories in accordance with an embodiment(s) herein;
Figure 17 illustrates a top down view of another probe configured such that its handle does not interfere with biopsy needle trajectories in accordance with an embodiment(s) herein;
Figure 18 illustrates a side view of the probe of Figure 16 or 17 showing several available biopsy needle trajectories in accordance with an embodiment(s) herein;
Figure 19 diagrammatically illustrates the imaging system and the biopsy instrument of Figure 5 in connection with a needle and transducer array tracking system in accordance with an embodiment(s) herein;
Figure 20 illustrates an example method in accordance with an embodiment(s) herein;
Figure 21 illustrates another example method in accordance with an embodiment(s) herein;
Figure 22 illustrates yet another example method in accordance with an embodiment(s) herein;
Figure 23 illustrates yet another example biopsy needle guide at a first position which provides a parallel trajectory in accordance with an embodiment(s) herein;
Figure 24 illustrates the biopsy guide of Figure 23 in which an actuator physically contacts and bends a needle or cannula to provide an angled trajectory in accordance with an embodiment(s) herein;
Figure 25 illustrates a top down view of another example probe configured with its handle angled with respect to the shaft such that it does not interfere with biopsy needle trajectories in accordance with an embodiment(s) herein; and
Figure 26 illustrates a side view of another example probe configured with its handle shifted up in the sagittal plane such that it does not interfere with biopsy needle trajectories in accordance with an embodiment(s) herein.

### DETAILED DESCRIPTION

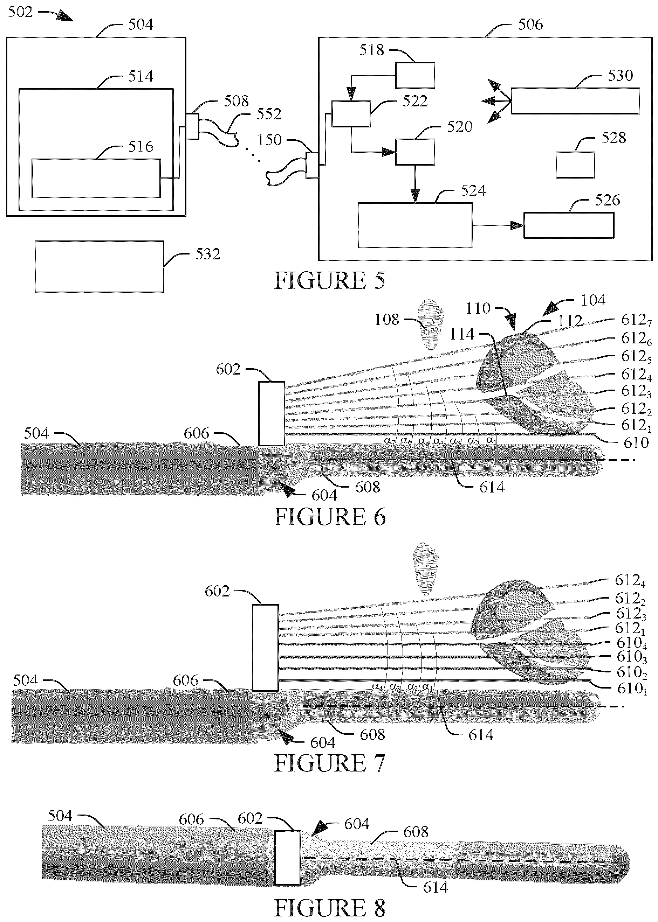

Figure 5 illustrates an example imaging system 502 such as an ultrasound imaging system / scanner. The imaging system 502 includes a probe 504 and a console 506, which interface with each other through suitable complementary hardware (e.g., cable connectors 508 and 510 and a cable 512 as shown, etc.) and/or wireless interfaces (not visible).

The probe 504 includes a transducer array 514 with one or more transducer elements 516. The one or more transducer elements 516 are arranged as a 1-D or 2-D, linear, curved and/or otherwise shaped, fully populated or sparse, etc. array. The elements 516 are configured to convert excitation electrical pulses into an ultrasound pressure field and convert a received ultrasound pressure field (an echo) into electrical (e.g., a radio frequency (RF)) signals.

The console 506 includes transmit circuitry (TX) 518 configured to generate the excitation electrical pulses and receive circuitry (RX) 520 configured to process the RF signals, e.g., amplify, digitize, and/or otherwise process the RF signals. The console 506 further includes a switch (SW) 522 configured to switch between the TX 518 and RX 520 for transmit and receive operations, e.g., by electrically connecting and electrically disconnecting the TX 518 and the RX 520.

The console 506 includes further an echo processor 524 configured to process the signal from the RX 520. For example, in one instance the echo processor 524 is configured to beamform (e.g., delay-and-sum) the signal to construct a scanplane of scanlines of data. The echo processor 524 can process data from 1-D and/or 2-D probes for 2-D, 3-D and/or 4-D applications. The echo processor 524 can be implemented by a hardware processor such as a central processing unit (CPU), a graphics processing unit (GPU), a microprocessor, etc.

The console 506 further includes a display 526. The output of the echo processor 524 is scan converted to the coordinate system of the display 526 and displayed as images via the display 526. In one instance, the scan converting includes changing the vertical and/or horizontal scan frequency of signal based on the display 526. The scan converter 128 can be configured to employ analog scan converting techniques and/or digital scan converting techniques.

The console 506 further includes a user interface 528, which includes one or more input devices (e.g., a button, a touch pad, a touch screen, etc.) and one or more output devices (e.g., a display screen, a speaker, etc.). The console 506 further includes a controller 530 configured to control one or more of the probe 504, the transmit circuitry 518, the receive circuitry 520, the switch 522, the echo processor 524, the display 526, the user interface 528, and/or one or more other components of the imaging system 502.

In the illustrated embodiment, the imaging system 502 is used in connection with a biopsy instrument 532. In this embodiment, the imaging system 502 is used to guide a needle of the biopsy instrument 532 to a target region within an object or subject where the biopsy instrument 532 is used to acquire a sample of the target region.

As described in greater detail below, a biopsy guide that provides for both parallel and angled trajectories can be employed with the probe 504 for prostrate biopsies. In one instance, this allows for taking biopsies more precisely at the peripheral zone while also being able to avoid the pubic archbone that can block access to the anterior apex. Also described in greater detail below, a handle of the probe 504 can be translated sideways so that it does not interfere with the sagittal plane. In one instance, this allows a needle trajectory path that was not previously possible. Also described in greater detail below, a tracking system can be employed with the imaging system 502. In one instance, this provides the flexibility of freehand biopsies with the accuracy of ultrasound-guided biopsies, making transperineal biopsies more versatile.

Figures 6 and 7 illustrate a non-limiting example of the probe 504 in connection with a guide 602 such as a biopsy needle guide. In this example, the guide 602 is supported at a region 604 between an elongate tubular handle 606 and an elongate tubular shaft 608 of the probe 504. In other embodiments, the guide 602 is supported on the handle 606, on the shaft 608, or a combination of the handle 606, the region 604 and/or the shaft 608. The guide 602, in one instance, is supported via a clamping and/or other device. In these examples, the guide 602 is a physical device pre-configured with a plurality of channels (not visible).

In Figure 6, the plurality of channels includes a single channel that provides a trajectory 610 that is parallel to an axis 614 of the shaft 608 and multiple channels that provide trajectories 612₁, 612₂, 612₃, 612₄, 612₅, 612₆, and 612₇ that are angled, with increasing angle α₁, α₂, α₃, α₄, α₅, α₆, and α₇, to the axis 614 of the shaft 608. In Figure 7, the plurality of channels includes multiple channels that provide trajectories 610₁, 610₂, 610₃ and 610₄ that are parallel, with increasing distance, to the axis 614 of the shaft 608 and multiple channels that provide trajectories 612₁, 612₂, 612₃ and 612₄ that are angled, with increasing angle α₁, α₂, α₃ and α₄, to the axis 614 of the shaft 608.

In both Figure 6 and Figure 7, the guide 602 provides trajectories to the peripheral zone 114 and the apex 110 of the anterior zone 112 of the prostate 104, including a trajectory to the apex 110 that avoids physical inference from the pubic archbone 108. In Figure 6, the former includes the parallel trajectory 610 and the latter includes the trajectory 612₇. In Figure 7, the former includes the parallel trajectory 610₁ and the latter includes the trajectory 612₄. Figure 8 shows a top down view where the guide 602 is centered in the region 604 on the axis 614.

In a variation, the guide 602 includes more, less and/or other channels for other trajectories. This may include interlaced channels for channels parallel and angled trajectories. This may also include angled trajectories that do not all increase with a distance from the axis 614. Furthermore, in another instance, the guide 602 is not centered in the region 604 on the axis 614. Other configurations are also contemplated herein.

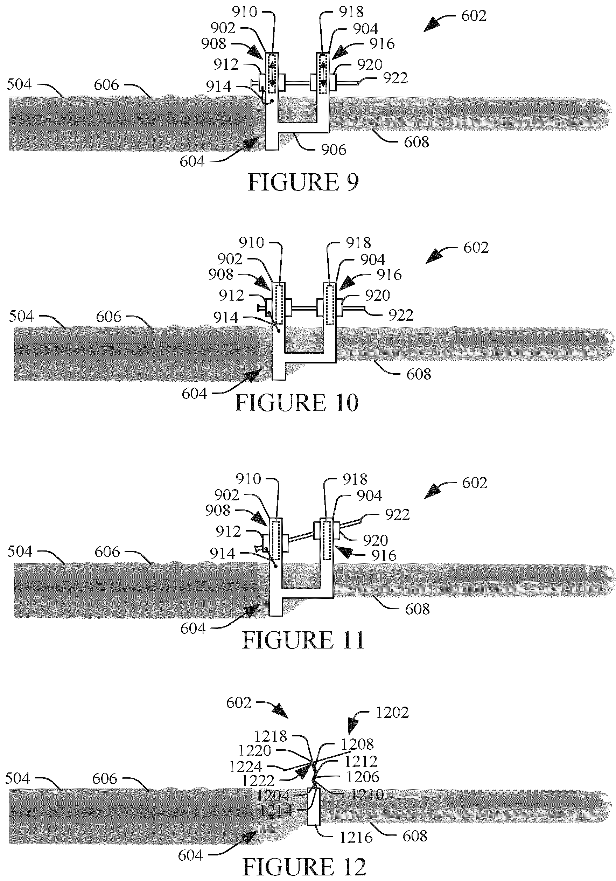

Figures 9-11 illustrate a non-limiting example of the guide 602. In this example, the guide 602 includes a vertical leg 902 having a length, a second vertical leg 906 having a length, and a horizontal leg 906. The length of the vertical leg 902 is greater than the length of the vertical leg 904. The vertical leg 902 clamps onto the region 604. The second vertical leg 904 is attached to the first vertical leg 902 through the horizontal leg 906.

The first vertical leg 902 includes an opening 908 in which a stationary part 910 of a linear bearing is disposed. A moveable part 912 of the linear bearing is engaged with the stationary part 910 and configured to move vertically within the first vertical leg 902. In this example, a limiter 914 (e.g., a wire, string, etc.) limits the vertical motion from an initial position in which the moveable part 912 is closest to the shaft 608 to a position at or less than a maximum position furthest away from the shaft 608.

The second vertical leg 904 includes an opening 916 in which a stationary part 918 of a linear bearing is disposed. A moveable part 920 of the linear bearing is engaged with the stationary part 918 and configured to move vertically within the second vertical leg 904. In this example, the moveable part 920 is free to move from the initial position in which the moveable part 920 is closest to the shaft 608 to the maximum position / the position furthest away from the shaft 608.

In this example, the moveable parts 912 and 920 are configured to support a trocar 922, which is configured to allow a biopsy needle to pass therethrough. In general, the trocar 922 is inserted through an incision in the perineum and provides access to the prostate with the biopsy needle without any further punctures through the skin, unless otherwise desired. In Figure 9, both of the moveable parts 912 and 920 are at their lowest positions and together provide a parallel trajectory (e.g., 610₁ in Figure 7) to the prostate.

In Figure 10, both moveable parts 912 and 920 are moved vertically to a same position at which the limiter 914 limits the moveable part 920 from further vertical motion. In this position, the moveable parts 912 and 920 together provide another parallel trajectory (e.g., 610₄ in Figure 7) to the prostate. In Figure 11, the moveable part 920 is moved vertically to its maximum allowable position. In this position, the moveable parts 912 and 920 together provide an angled trajectory (e.g., 612₄ in Figure 7) to the prostate.

In one instance, both moveable parts 912 and 920 move together between the lower limit and the limit of the limiter 914. In another instance, the moveable parts 912 and 920 move independently between the lower limit and the limit of the limiter 914. The moveable parts 912 and 920 can be moved by way of linear translation of a handle, a rotational-to-linear translation of a rotary dial, a pivotal-to-linear translation of a lever, and/or other mechanism. A locking mechanism can be used to hold them in place.

In a variation, both moveable parts 912 and 920 of the bearings are configured to hold the biopsy needle in alternative to or in addition to the trocar 922. In this configuration, the biopsy needle is slidable within the moveable parts 912 and 920 to allow the biopsy needle to be advanced to the prostate to take a sample and retracted from the prostate after a sample is taken. Furthermore, other guide shapes, locations of attachment, and/or moving mechanisms are contemplated herein.

Figure 12 illustrate another non-limiting example of the guide 602. In this example, the guide 602 attaches to the shaft 608 and includes a mechanical arm 1202 with multiple segments 1204, 1206 and 1208 which are connected together by joints 1210 and 1212 which are configured to move in one direction along one axis, within a plane defined by two axes, or within a volume defined by three axes.

A first end 1214 of the mechanical arm 1202 is affixed to a clamp 1216 attached to the shaft 608. A second end 1218 at an opposing end of the mechanical arm 1202 includes a member 1220 with a guide hole 1222 configured to allow a biopsy needle 1224 to pass therethrough. In general, this embodiment allows free-hand positioning of biopsy needle 1224 under the guidance of the mechanical arm 1202. In a variation, there are more or less segments and/or joints.

With the configurations of Figures 9-12, a biopsy procedure can be performed with a single perforation point in the perineum to obtain multiple samples, e.g., from the peripheral zone 114 to the anterior apex 110, by tilting / pivoting the guide 602. Alternatively, the biopsy procedure can be performed through multiple perforation points in the perineum, each corresponding to a different trajectory to acquire a different sample.

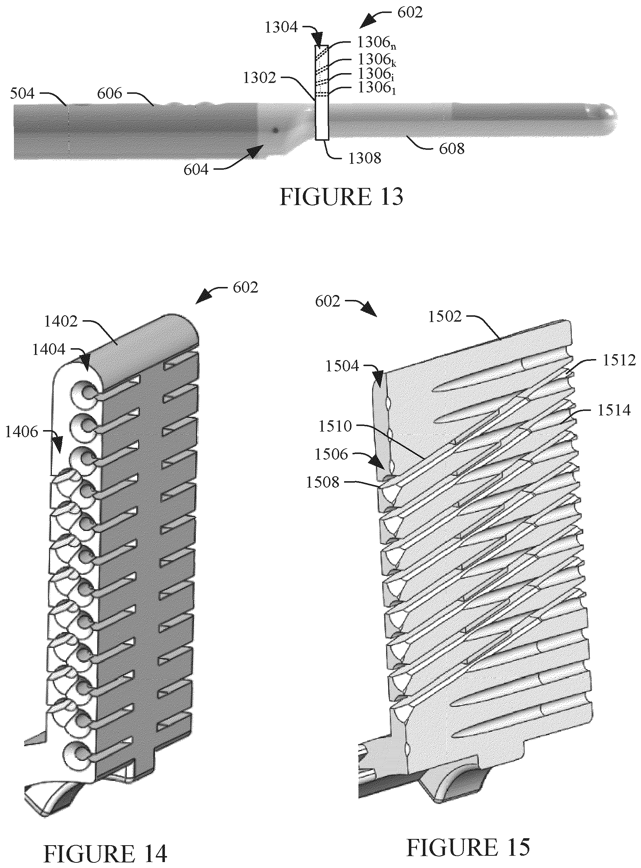

Figure 13 illustrates yet another non-limiting examples of the guide 602. In Figure 13 the guide 602 includes a block 1302 with a column 1304 of predetermined channels 1306₁, ..., 1306ᵢ, ..., 1306ₖ, ..., 1306ₙ, where n is a positive integer, and an end 1308 that clamps to the shaft 608. In this example, the channel 1306₁ provides a parallel trajectory and the channels 1306ᵢ, ..., 1306ₖ, ..., 1306ₙ provide angled trajectories.

Figure 14 illustrates yet another non-limiting examples of the guide 602. In Figure 14 the guide 602 includes a block 1402 with two columns 1404 and 1406 of predetermined channels. In this example, the column 1404 includes channels that provide parallel biopsy needle trajectories and the column 1406 includes channels that provide angled biopsy needle trajectories.

Figure 15 illustrates still another non-limiting examples of the guide 602. In Figure 15 the guide 602 includes a block 1502 with a single column 1504 of predetermined channels. In this example, each channel 1506 includes a single entry point 1508 and a single path 1510 partway through the guide 602 and then alternative branches 1512 and 1514 thereafter, where the branch segment 1512 provides a parallel biopsy needle trajectory, and the branch 1514 provides an angled biopsy needle trajectory.

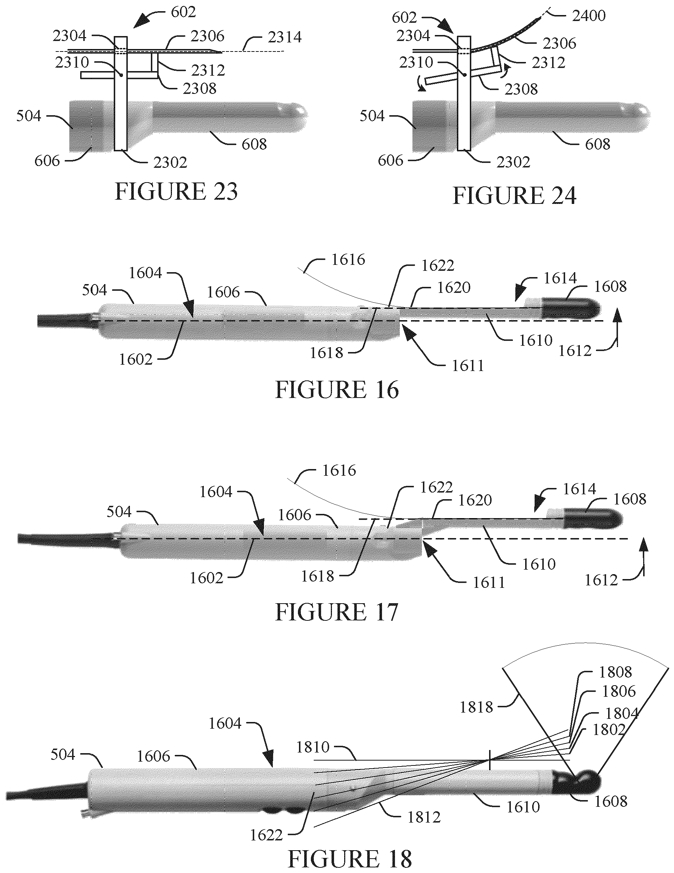

Figures 23 and 24 illustrate yet another non-limiting examples of the guide 602. In this example, the guide 602 includes a support 2302 configured to couple to the probe 504, an access channel 2304 is configured to support a needle instrument 2306 (e.g., a needle or a cannula), and an actuator 2308 is pivotably mounted to the support 2302 at a pivot point 2310. In this example, the actuator 2308 is a cuboid shaped with a nub 2312 at an end proximal to the shaft 608. Other shapes are also contemplated herein. In the non-actuating state (Figure 23), the nub 2312 is just below or just touching the needle instrument 2306 allowing for a parallel trajectory 2314 with respect to the shaft 608.

In an actuating state (Figure 24), the actuator 2308 is pivoted about the pivot point 2310 in a direction towards the handle 606. As a result, the opposing end pivots about the pivot point 2310 away from the shaft 608, which causes the nub 2408 to physically contact the needle instrument 2406 (if not already in physical contact) and urge the needle instrument 2306 away from the shaft 608, which causes the needle instrument 2306 to bend or elastically deform, creating an angled trajectory 2400. The illustrated trajectory is not limiting, and the actuator 2308 can be pivoted more or less to create other angled trajectories.

With the configurations of Figures 6-15, 23 and 24, in one instance, biopsies can be taken more precisely at the peripheral zone 114 (Figure 1), which is where most cancers occur, while also being able to avoid the pubic archbone (Figure 1) that can block access to the anterior apex 110 (Figure 1).

Figures 16-18 illustrate another embodiment of the probe 504. In this example, the probe 504 has a long axis 1602 and a top side 1604 that extends along the long axis 1602. The probe 504 further includes an elongate tubular handle 1606, a head 1608, and an elongate tubular shaft 1610 disposed between the elongate tubular handle 1606 and the head 1608. The transducer array 514 (Figure 5) is disposed in the head 1608 and is configured to transmit in a sagittal plane (i.e. the plane cutting through a center line 1618 of the shaft 1610) extending in a direction only out from the top side 1604. The elongate tubular shaft 1610 is coupled to the elongate tubular handle 1606 linearly offset from a center 1611 of the elongate tubular handle 1606 in a direction transverse 1612 to the long axis 1602 and the sagittal plane.

Figures 16 and 17 illustrate a top-down view of the probe 504. The elongate tubular shaft 1610 includes a recessed region 1614 to provide space for a needle 1616 to be advanced along the center line 1618 of the shaft 1610. In Figure 16, a surface 1620 of the recessed region 1614 is flush with a surface of a side 1622 of the handle 1606 forming a planar surface therewith. In Figure 17, the surface 1620 of the recessed region 1614 protrudes out relative to the surface of a side 1622 of the handle 1606. With both Figures 16 and 17, the needle 1616 can be advanced from below (or above) the handle 1606 to above (or below) the handle 1606 without any physical interference from the probe 504.

This allows the biopsy trajectory paths to be positioned around one needle perforation site and the entire prostate can be biopsied. An example is shown in Figure 18, which shows a side view and several needle trajectories 1802, 1804, 1806 and 1808 between a parallel trajectory 1810 above the probe 504 and an angled trajectory 1812 from below the probe 504. This probe configuration mitigates the physical interference (Figures 2-4) of the side 1622 of the handle 1606 of the probe 504, which interferes with such needle trajectories. Figure 18 also shows an image plane 1818 extending from the center line 1618 of the shaft 1610 in the direction only out from the top side 1604 and the sagittal image plane 1818.

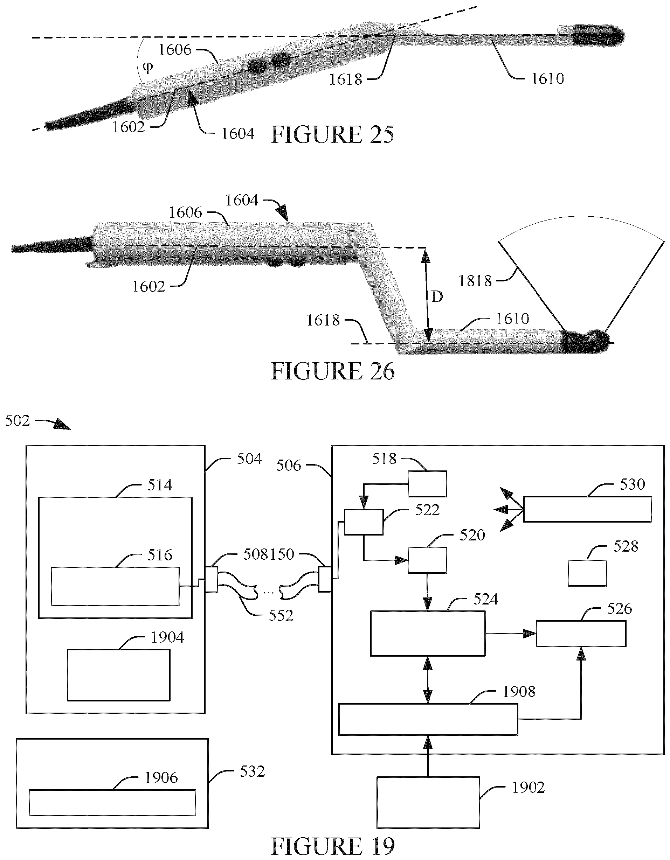

Figure 25 shows a variation of the probe 504 of Figures 16-18 from the top-down view of the probe 504. In this variation, the handle 1606 is instead angled in a plane extending through the handle 1606 and the shaft 1610 that is transverse to the sagittal image plane 1818 of Figure 18 and sideways with respect to the shaft 1610 by an angle φ. Similar to Figures 16 and 17, this allows the trajectories 1802, 1804, 1806 and 1808 shown in Figure 18 between the parallel trajectory 1810 above the probe 504 and the angled trajectory 1812 from below the probe 504. In another variation, the handle 1606 can be angled as shown in Figure 25 and the shaft 1610 can be offset as shown in Figure 16 or Figure 17.

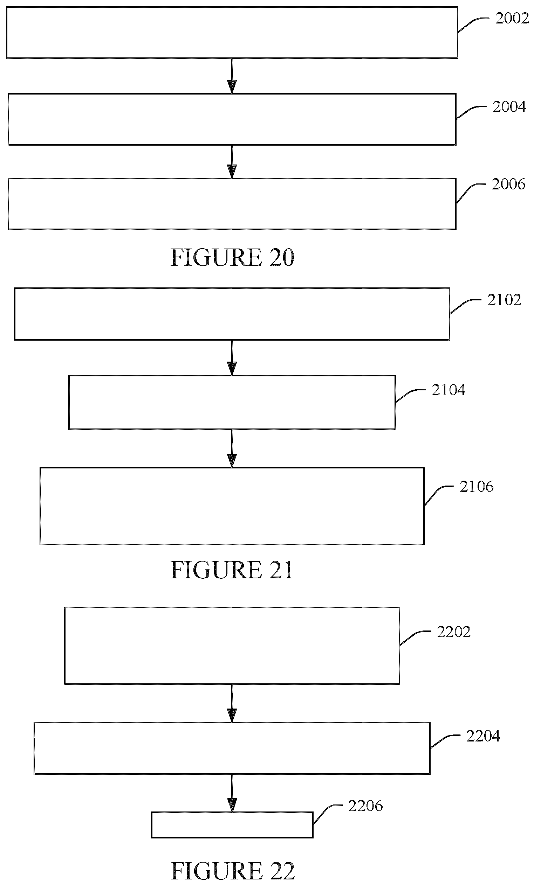

Figure 26 shows another variation of the probe 504 of Figures 16-18 from the side view. In this variation, the handle 1606 is instead shifted up in the sagittal plane in a direction of the image plane 1818 by a distance "d". Similar to Figures 16 and 17, this allows the trajectories 1802, 1804, 1806 and 1808 shown in Figure 18 between the parallel trajectory 1810 above the probe 504 and the angled trajectory 1812 from below the probe 504. In another variation, the handle 1606 can be shifted as shown in Figure 20 and angled as shown in Figure 26 and/or the shaft 1610 can be offset as shown in Figure 16 or Figure 17.

In general, with the configurations of Figures 16-18, 25 and 26, the elongate tubular handle 1606 is not both centered on the elongate tubular shaft 1610 (like in Figures 2-4) and shifted down in the sagittal image plane 1818 away from the top side 1604 of the elongate tubular shaft 1610. In addition, the guide 602 of Figures 5-15, 23 and 24 can be employed with the probe of Figures 16-18, 25 and 26.

Figure 19 illustrates a variation of the imaging system 502. In this variation, the probe 504 is configured to acquire three-dimensional volumetric data, the system 502 includes a probe and biopsy instrument tracking system 1902, the probe 504 further includes an internal and/or external tracking device(s) 1904, and the biopsy instrument 532 includes an internal and/or external tracking device(s) 1906. The probe and biopsy instrument tracking system 1902 tracks a spatial location of the biopsy instrument 532 relative to the probe 504 and produces a tracking signal / tracking data indicative thereof. The probe 504 is used to perform a free-hand or guided prostate biopsy.

The console 506 further includes an image processor 1908. The image processor 1908 utilize the tracking signal / tracking data to register spatial coordinate systems of the probe 504 and the biopsy instrument 532 and identify a cross-sectional plane in the 3-D ultrasound data that shows a needle of the biopsy instrument 532 and its trajectory, and this image is displayed via the display 526. Where a pre-procedure scan (e.g., MRI, CT, etc.) is available and a target is located in the resulting 3-D data, the image processor 1908 superimposes and registers the pre-procedure 3-D data and the 3-D ultrasound data and selects and displays a plane that shows the needle, its trajectory and the target. This variation provides flexibility of freehand biopsies along with a visual guide and accurate tracking.

Suitable tracking includes electromagnetic, optical, mechanical, etc. With electromagnetic tracking, in one instance, tracking coils are included with both the transducer 514 and a needle of the biopsy instrument 532. The tracking system 1902 measures a magnetic field strength of the coils, which depends on a distance and direction of the coils to the tracking system 1902, and the strength and direction is used to determine location and orientation.

With optical tracking, in one instance, fiducial targets are placed on both the transducer 514 and a needle of the biopsy instrument 532. The tracking system 1902 includes an optical device such as a video camera that records the spatial orientation of the optical elements to determine location and orientation.

With mechanical tracking, in one instance, a passive µ-robot arm (e.g., with 5-6 degrees of freedom) is mounted on the probe 504. The robot arm holds a needle guide that can be turned freely, e.g., in two angles (such as elevation and azimuth), and a tip of the guide can be placed freely at any position within the reach of the arm. Encoders or the like disposed at joints of the robot arm provide position signal, which the tracking system 1902 utilizes to determine the spatial relationship between the transducer array 514 if the probe 504 and the needle the biopsy instrument 532.

Suitable tracking devices and systems are discussed in Birkfellner et al., "Tracking Devices," In: Peters T., Cleary K. (eds) Image-Guided Interventions. Springer, Boston, MA, 2008. Suitable tracking systems are described in U.S. patent application US 2010/0298712 A1, filed February 10, 2010, and entitled "Ultrasound Systems Incorporating Position Sensors and Associated Method," and U.S. patent US 8,556,815 B2, filed May 6, 2010, and entitled "Freehand Ultrasound Imaging Systems and Methods for Guiding Elongate Instruments". Another example is discussed in US 7,835,785 B2, filed October 4, 2005, and entitled "DC Magnetic-Based Position and Orientation Monitoring system for Tracking Medical Instruments." Other approaches are also contemplated herein.

With Figures 6-19 and 23-26, a biopsy needle can be inserted directly through the perineum and/or through a hollow access channel of an access device (e.g., a trocar) that is inserted directly through the perineum.

FIGURE 20 illustrates an example method in accordance with an embodiment herein.

It is to be appreciated that the order of the below acts is not limiting, and in other embodiments, there may be more, less and/or different acts.

At 2002, an ultrasound imaging probe with a guide, which is configured for both parallel and angled biopsy needle trajectories, attached thereto is positioned for a perineal biopsy.

At 2004, the guide guides a biopsy needle along a parallel trajectory to a biopsy site (e.g., the peripheral region of the prostrate) where a sample is acquired.

At 2006, the guide guides the biopsy needle along an angled trajectory to another biopsy site (e.g., the apex of the anterior region of the prostrate) where a sample is acquired.

Optionally, the guide is used to guide the biopsy needle along one or more other parallel and/or angled trajectories to other biopsy site where samples are acquired.

FIGURE 21 illustrates an example method in accordance with an embodiment herein.

It is to be appreciated that the order of the below acts is not limiting, and in other embodiments, there may be more, less and/or different acts.

At 2102, an ultrasound imaging probe with a shaft offset relative of a handle of the probe so as not to physically interfere with biopsy trajectories in a sagittal plane is obtained.

At 2104, a biopsy needle is advanced along the probe via a parallel (or angled) trajectory to a biopsy site where a sample is acquired.

At 2106, the biopsy needle is pivoted, without physical interference of the probe with the trajectory, and advanced along the probe via an angled (parallel) trajectory to a biopsy site where a sample is acquired.

FIGURE 22 illustrates an example method in accordance with an embodiment herein.

It is to be appreciated that the order of the below acts is not limiting, and in other embodiments, there may be more, less and/or different acts.

At 2202, a tracking system detects a spatial position of a biopsy needle relative to an ultrasound probe and generates a signal indicative thereof.

At 2204, an image processor selects a 2-D plane of 3-D ultrasound image date that includes an access point and a trajectory of the biopsy needle.

At 2206, the selected 2-D plane is displayed.

Optionally, the selected plane also includes a biopsy target.

## Claims

1. A system (502) for ultrasound-guided transperineal and/or transrectal biopsy, comprising:
a needle biopsy guide (602), including:
a first portion configured to couple to an elongate ultrasound imaging probe (504);
a hollow channel configured to guide a needle or a cannula along a trajectory that is parallel to a long axis of the elongated ultrasound imaging probe and along a trajectory that is angled to the long axis;
a support configured to couple to a probe;
an access channel configured to support the needle or the cannula;
an actuator with a nub on one end; and
a pivot point,
wherein the actuator is pivotably mounted to the support at the pivot point and configured to move between a position in which the needle or the cannula traverses a trajectory parallel to the long axis of the elongated ultrasound imaging probe and a position in which the nub elastically deforms the needle or the cannula to traverse a trajectory angled to the long axis of the elongated ultrasound imaging probe.

2. The system of claim 1, wherein the hollow channel includes a common first portion and a second portion with different branching second portions, wherein one of the branching portions is for the parallel trajectory and another of the branching portions is for the angled trajectory.

3. The system of claim 1, the needle biopsy guide further comprising:
a first vertical leg, including:
an opening;
a first portion of a bearing disposed in the opening; and
a second portion of the bearing configured to move vertically within the first portion of the bearing, wherein the second portion is configured to support the needle or the cannula;
a second vertical leg, including:
an opening;
a first portion of a bearing disposed in the opening; and
a second portion of the bearing configured to move vertically within the first portion of the bearing, wherein the second portion is configured to support the needle or the cannula; and
a limiter,
wherein the limiter is configured to limit travel of the second portion of the bearing of the first vertical leg, and the second portions of the first and second vertical legs are configured to travel between a position providing the parallel trajectory and a position providing the angled trajectory.

4. The system of claim 1, the needle biopsy guide further comprising:
a mechanical arm that supports the hollow channel and that is configured to move with a plurality of degrees of freedom to guide the needle or the cannula along the parallel trajectory and the angled trajectory.

5. The system of claim 1,
an elongate ultrasound imaging probe (504) with a long axis and a top side that extends along the long axis, including:
an elongate tubular handle (606; 1606);
a head (1608);
an elongate tubular shaft (608; 1610) disposed between the elongate tubular handle and the head; and
a transducer array (514) disposed in the head and configured to transmit in a sagittal plane only in a direction extending out from the top side;
wherein the elongate tubular handle is not centered on the elongate tubular shaft and is not shifted down in the sagittal plane away from the top.

6. The system of claim 5, wherein the elongate tubular shaft is linearly offset from a center line of the elongate tubular handle in a direction transverse to the long axis of the handle and the sagittal plane.

7. The system of claim 6, wherein the elongate tubular shaft includes a recessed region (1614) with a recessed surface located along a central axis of the elongate tubular shaft, and the surface is either planar with a side of the handle or extends out passed the side of the handle.

8. The system of claim 5, wherein the elongate tubular handle is angled in a direction transverse to the long axis of the handle and the sagittal plane with respect to the elongate tubular shaft.

9. The system of claim 5, wherein the elongate tubular handle is shifted up in the sagittal plane away from the elongate tubular shaft and the head.

10. The system of claim 1, further comprising:
an elongate ultrasound imaging probe (504) including a first tracking device (1904);
a needle biopsy instrument (532) including a second tracking device (1906);
a tracking system (1902) configured to track a spatial location of the needle biopsy instrument and the elongate ultrasound imaging probe based on signals from the first and second tracking devices;
an image processor (1908) configured select a plane from volumetric image data generated with ultrasound echoes detected by a transducer array of the elongate ultrasound imaging probe based on the tracked spatial locations, wherein the plane includes an entry point of the needle biopsy instrument in an object and at least a trajectory of the needle biopsy instrument in the object; and
a display (526) configured to display the selected plane.

11. The system of claim 10, wherein the selected plane further includes a biopsy target region of the object.

12. The system of claim 10, wherein the image processor fuses the selected plane with previously acquired computed tomography or magnetic resonance imaging volumetric image data.

13. The system of claim 10, wherein the tracking system is one of an electromagnetic, optical or mechanical tracking system.

## Patentansprüche

1. System (502) für ultraschallgeführte transperineale und/oder transrektale Biopsie, umfassend:
eine Nadelbiopsieführung (602), die Folgendes einschließt:
einen ersten Abschnitt, der so konfiguriert ist, dass er mit einer länglichen Ultraschall-Bildgebungssonde (504) gekoppelt wird;
einen hohlen Kanal, der so konfiguriert ist, dass er eine Nadel oder Kanüle entlang einer Trajektorie führt, die parallel zu einer Längsachse der länglichen Ultraschall-Bildgebungssonde und entlang einer Trajektorie, die zur Längsachse abgewinkelt ist, verläuft;
einen Träger, der so konfiguriert ist, dass er mit einer Sonde gekoppelt wird;
einen Zugangskanal, der so konfiguriert ist, dass er die Nadel oder die Kanüle trägt;
eine Betätigungsvorrichtung mit einem Zapfen an einem Ende; und
einen Lagerpunkt,
wobei die Betätigungsvorrichtung am Lagerpunkt schwenkbar an der Stütze montiert ist und so konfiguriert ist, dass sie sich zwischen einer Position, in der die Nadel oder die Kanüle eine Trajektorie parallel zur Längsachse der länglichen Ultraschall-Bildgebungssonde durchläuft, und einer Position, in der der Zapfen die Nadel oder die Kanüle elastisch verformt, um eine zur Längsachse der länglichen Ultraschall-Bildgebungssonde abgewinkelte Trajektorie zu durchlaufen, bewegt.

2. System nach Anspruch 1, wobei der hohle Kanal einen gemeinsamen ersten Abschnitt und einen zweiten Abschnitt mit verschiedenen verzweigten zweiten Abschnitten einschließt, wobei einer der verzweigten Abschnitte für die parallele Trajektorie dient und ein anderer der verzweigten Abschnitte für die abgewinkelte Trajektorie dient.

3. System nach Anspruch 1, wobei die Nadelbiopsieführung weiter Folgendes umfasst:
einen ersten vertikalen Schenkel, der Folgendes einschließt:
eine Öffnung;
einen ersten Abschnitt eines in der Öffnung angeordneten Lagers; und
einen zweiten Abschnitt des Lagers, der so konfiguriert ist, dass er sich innerhalb des ersten Abschnitts des Lagers vertikal bewegt, wobei der zweite Abschnitt so konfiguriert ist, dass er die Nadel oder die Kanüle trägt;
einen zweiten vertikalen Schenkel, der Folgendes einschließt:
eine Öffnung;
einen ersten Abschnitt eines in der Öffnung angeordneten Lagers; und
einen zweiten Abschnitt des Lagers, der so konfiguriert ist, dass er sich innerhalb des ersten Abschnitts des Lagers vertikal bewegt, wobei der zweite Abschnitt so konfiguriert ist, dass er die Nadel oder die Kanüle trägt; und
einen Begrenzer,
wobei der Begrenzer so konfiguriert ist, dass er Verfahren des zweiten Abschnitts des Lagers des ersten vertikalen Schenkels begrenzt, und die zweiten Abschnitte des ersten und zweiten vertikalen Schenkels so konfiguriert sind, dass sie zwischen einer Position, die die parallele Trajektorie bereitstellt, und einer Position, die die abgewinkelte Trajektorie bereitstellt, verfährt.

4. System nach Anspruch 1, wobei die Nadelbiopsieführung weiter Folgendes umfasst:
einen mechanischen Arm, der den hohlen Kanal trägt, und der so konfiguriert ist, dass er sich mit einer Vielzahl von Freiheitsgraden bewegt, um die Nadel oder die Kanüle entlang der parallelen Trajektorie und der abgewinkelten Trajektorie zu führen.

5. System nach Anspruch 1, wobei
eine längliche Ultraschall-Bildgebungssonde (504) mit einer Längsachse und einer Oberseite, die sich entlang der Längsachse erstreckt, Folgendes einschließt:
einen länglichen röhrenförmigen Griff (606; 1606);
einen Kopf (1608);
einen länglichen rohrförmigen Schaft (608; 1610), der zwischen dem länglichen rohrförmigen Griff und dem Kopf angeordnet ist; und
eine Wandleranordnung (514), die im Kopf angeordnet ist und so konfiguriert ist, dass sie in einer Sagittalebene nur in einer Richtung überträgt, die sich von der Oberseite nach außen erstreckt;
wobei der längliche rohrförmige Griff nicht auf dem länglichen rohrförmigen Schaft zentriert ist und nicht in der Sagittalebene von der Oberseite weg nach unten verschoben ist.

6. System nach Anspruch 5, wobei der längliche rohrförmige Schaft von einer Mittellinie des länglichen rohrförmigen Griffs in einer Richtung quer zur Längsachse des Griffs und zur Sagittalebene linear versetzt ist.

7. System nach Anspruch 6, wobei der längliche rohrförmige Schaft einen vertieften Bereich (1614) mit einer vertieften Fläche einschließt, die entlang einer Mittelachse des länglichen rohrförmigen Schafts gelegen ist, und wobei die Fläche entweder plan mit einer Seite des Griffs ist oder sich über die Seite des Griffs hinaus erstreckt.

8. System nach Anspruch 5, wobei der längliche rohrförmige Griff in einer Richtung quer zur Längsachse des Griffs und zur Sagittalebene in Bezug auf den länglichen rohrförmigen Schaft abgewinkelt ist.

9. System nach Anspruch 5, wobei der längliche rohrförmige Griff in der Sagittalebene vom länglichen rohrförmigen Schaft und dem Kopf weg nach oben verschoben ist.

10. System nach Anspruch 1, weiter umfassend:
eine längliche Ultraschall-Bildgebungssonde (504), die eine erste Nachverfolgungsvorrichtung (1904) einschließt;
ein Nadelbiopsieinstrument (532), das eine zweite Nachverfolgungsvorrichtung (1906) einschließt;
ein Nachverfolgungssystem (1902), das so konfiguriert ist, dass es eine räumliche Position des Nadelbiopsieinstruments und der länglichen Ultraschall-Bildgebungssonde basierend auf Signalen von der ersten und zweiten Nachverfolgungsvorrichtung verfolgt;
einen Bildprozessor (1908), der so konfiguriert ist, dass er eine Ebene aus volumetrischen Bilddaten auswählt, die mit Ultraschallechos erzeugt wurden, die von einer Wandleranordnung der länglichen Ultraschall-Bildgebungssonde erfasst wurden, basierend auf den verfolgten räumlichen Positionen, wobei die Ebene einen Eintrittspunkt des Nadelbiopsieinstruments in ein Objekt und mindestens eine Trajektorie des Nadelbiopsieinstruments in dem Objekt einschließt; und
eine Anzeige (526), die so konfiguriert ist, dass sie die ausgewählte Ebene anzeigt.

11. System nach Anspruch 10, wobei die ausgewählte Ebene weiter einen Biopsiezielbereich des Objekts einschließt.

12. System nach Anspruch 10, wobei der Bildprozessor die ausgewählte Ebene mit zuvor erfassten Bilddaten aus Computertomographie- oder Magnetresonanz-Bildgebung verschmilzt.

13. System nach Anspruch 10, wobei es sich bei dem Nachverfolgungssystem um ein elektromagnetisches, optisches oder mechanisches Nachverfolgungssystem handelt.

## Revendications

1. Système (502) pour une biopsie transpérinéale et/ou transrectale guidée par ultrasons, comprenant :
un guide de biopsie à l'aiguille (602), incluant :
une première partie configurée pour se coupler à une sonde d'imagerie ultrasonore allongée (504) ;
un canal creux configuré pour guider une aiguille ou une canule le long d'une trajectoire qui est parallèle à un axe longitudinal de la sonde d'imagerie ultrasonore allongée et le long d'une trajectoire qui est inclinée par rapport à l'axe longitudinal ;
un support configuré pour se coupler à une sonde ;
un canal d'accès configuré pour supporter l'aiguille ou la canule ;
un actionneur avec un renflement sur une extrémité ; et
un point de pivot,
dans lequel l'actionneur est monté de manière pivotante sur le support au niveau du point de pivot et configuré pour se déplacer entre une position dans laquelle l'aiguille ou la canule suit une trajectoire parallèle à l'axe longitudinal de la sonde d'imagerie ultrasonore allongée et une position dans laquelle le renflement déforme élastiquement l'aiguille ou la canule pour suivre une trajectoire inclinée par rapport à l'axe longitudinal de la sonde d'imagerie ultrasonore allongée.

2. Système selon la revendication 1, dans lequel le canal creux inclut une première partie commune et une seconde partie avec des secondes parties de ramification différentes, dans lequel l'une des parties de ramification est destinée à la trajectoire parallèle et une autre des parties de ramification est destinée à la trajectoire inclinée.

3. Système selon la revendication 1, le guide de biopsie à l'aiguille comprenant en outre :
une première branche verticale, incluant :
une ouverture ;
une première partie d'un palier disposée dans l'ouverture ; et
une seconde partie du palier configurée pour se déplacer verticalement à l'intérieur de la première partie du palier, dans lequel la seconde partie est configurée pour supporter l'aiguille ou la canule ;
une seconde branche verticale, incluant :
une ouverture ;
une première partie d'un palier disposée dans l'ouverture ; et
une seconde partie du palier configurée pour se déplacer verticalement à l'intérieur de la première partie du palier, dans lequel la seconde partie est configurée pour supporter l'aiguille ou la canule ; et
un limiteur,
dans lequel le limiteur est configuré pour limiter un déplacement de la seconde partie du palier de la première branche verticale et les secondes parties des première et seconde branches verticales sont configurées pour se déplacer entre une position fournissant la trajectoire parallèle et une position fournissant la trajectoire inclinée.

4. Système selon la revendication 1, le guide de biopsie à l'aiguille comprenant en outre :
un bras mécanique qui supporte le canal creux et qui est configuré pour se déplacer avec une pluralité de degrés de liberté pour guider l'aiguille ou la canule le long de la trajectoire parallèle et de la trajectoire inclinée.

5. Système selon la revendication 1,
une sonde d'imagerie ultrasonore allongée (504) avec un axe longitudinal et un côté supérieur qui s'étend le long de l'axe longitudinal, incluant :
une poignée tubulaire allongée (606 ; 1606) ;
une tête (1608) ;
une tige tubulaire allongée (608 ; 1610) disposée entre la poignée tubulaire allongée et la tête ; et
un réseau de transducteurs (514) disposé dans la tête et configuré pour émettre dans un plan sagittal uniquement dans une direction s'étendant à partir du côté supérieur ;
dans lequel la poignée tubulaire allongée n'est pas centrée sur la tige tubulaire allongée et n'est pas décalée vers le bas dans le plan sagittal en s'éloignant du haut.

6. Système selon la revendication 5, dans lequel la tige tubulaire allongée est décalée linéairement par rapport à une ligne centrale de la poignée tubulaire allongée dans une direction transversale à l'axe longitudinal de la poignée et au plan sagittal.

7. Système selon la revendication 6, dans lequel la tige tubulaire allongée inclut une région en creux (1614) avec une surface en creux située le long d'un axe central de la tige tubulaire allongée et la surface est soit plane avec un côté de la poignée, soit s'étend au-delà du côté de la poignée.

8. Système selon la revendication 5, dans lequel la poignée tubulaire allongée est inclinée dans une direction transversale à l'axe longitudinal de la poignée et au plan sagittal par rapport à la tige tubulaire allongée.

9. Système selon la revendication 5, dans lequel la poignée tubulaire allongée est décalée vers le haut dans le plan sagittal en s'éloignant de la tige tubulaire allongée et de la tête.

10. Système selon la revendication 1, comprenant en outre :
une sonde d'imagerie ultrasonore allongée (504) incluant un premier dispositif de suivi (1904) ;
un instrument de biopsie à l'aiguille (532) incluant un second dispositif de suivi (1906) ;
un système de suivi (1902) configuré pour suivre un emplacement spatial de l'instrument de biopsie à l'aiguille et de la sonde d'imagerie ultrasonore allongée sur la base de signaux provenant des premier et second dispositifs de suivi ;
un processeur d'images (1908) configuré pour sélectionner un plan à partir de données d'images volumétriques générées avec des échos ultrasonores détectés par un réseau de transducteurs de la sonde d'imagerie ultrasonore allongée sur la base des emplacements spatiaux suivis, dans lequel le plan inclut un point d'entrée de l'instrument de biopsie à l'aiguille dans un objet et au moins une trajectoire de l'instrument de biopsie à l'aiguille dans l'objet ; et
un afficheur (526) configuré pour afficher le plan sélectionné.

11. Système selon la revendication 10, dans lequel le plan sélectionné inclut en outre une région cible de biopsie de l'objet.

12. Système selon la revendication 10, dans lequel le processeur d'images fusionne le plan sélectionné avec des données d'images volumétriques de tomodensitométrie ou d'imagerie par résonance magnétique acquises précédemment.

13. Système selon la revendication 10, dans lequel le système de suivi est l'un parmi un système de suivi électromagnétique, optique ou mécanique.
